# EUROPÄISCHE PATENTSCHRIFT

(11) **EP 2 384 699 B1**
(45) Veröffentlichungstag und Bekanntmachung des Hinweises auf die Patenterteilung: **12.12.2012**
(21) Anmeldenummer: 10004776.0
(22) Anmeldetag: 06.05.2010
(51) Int. Cl.: A61B 5/151

(54) **Lanzettenmagazin und Verfahren zu seiner Herstellung**
Lancet cartridge and method for its production
Cartouche de lancettes et son procédé de fabrication

(43) Veröffentlichungstag der Anmeldung: 09.11.2011
(73) Patentinhaber: Roche Diagnostics GmbH, 68305 Mannheim (DE); F.Hoffmann-La Roche AG, 4070 Basel (CH)
(72) Erfinder: Harttig, Herbert, 67434 Neustadt (DE)
(74) Vertreter: Mommer, Niels

(56) Entgegenhaltungen:
- EP-B1- 0 951 939
- DE-A1-102005 003 789
- US-A- 5 070 886

## Beschreibung

Die Erfindung geht aus von einem Lanzettenmagazin mit den im Oberbegriff des Anspruchs 1 angegebenen Merkmalen. Ein derartiges Lanzettenmagazin hat ein Gehäuse und mehrere Lanzetten, die jeweils in einer sterilen Kammer des Gehäuses eingeschlossen sind, wobei die Kammern jeweils eine Stichöffnung aufweisen, die mit einer Folie, beispielsweise aus Kunststoff, Metall oder Papier, verschlossen ist. Ein solches Lanzettenmagazin ist beispielsweise aus der EP 0 951 939 B1 bekannt.

Bei dem bekannten Lanzettenmagazin sind die Lanzetten zusammen mit Trockenmittel in Gehäusekammern angeordnet, deren Öffnungen mit Folie feuchtigkeitsdicht versiegelt sind. Lanzetten werden in einem solchen Magazin durch intensive Strahlungseinwirkung sterilisiert oder mit sehr großem Aufwand unter sterilen Bedingungen in das Magazin eingebracht und dieses anschließend versiegelt. Lanzettenmagazine werden für Stechgeräte benötigt, mit denen Körperflüssigkeitsproben, in der Regel Blut und/oder interstitielle Flüssigkeit, zur Messung einer Analytkonzentration gewonnen werden. Derartige Stechgeräte und dazu passende Lanzettenmagazine werden beispielsweise von Diabetikern benötigt, die mehrmals täglich ihren Blutzuckerspiegel überprüfen müssen.

Lanzettenmagazine enthalten steril verpackt jeweils mehrere Lanzetten, die mit einem Stechgerät nacheinander verwendet werden können. Lanzettenmagazine ermöglichen einen hohen Benutzerkomfort, da sie mit einfachen Handgriffen in ein Stechgerät eingesetzt und erst nach Gebrauch aller darin enthaltenen Lanzetten ausgetauscht werden müssen.

Neben einfachen Lanzetten, die lediglich zur Erzeugung einer Stichwunde dienen, sind auch Lanzetten bekannt, die eine Probenaufnahmeeinrichtung, beispielsweise einen Kapillarkanal aufweisen. Derartige Lanzetten ermöglichen einen besonders hohen Benutzerkomfort, da eine separate Handlung des Benutzers zur Aufnahme einer Probe nicht erforderlich ist. Beim Einstich in den Körper eines Patienten nimmt eine solche Lanzette von selbst eine Körperflüssigkeitsprobe auf, die dann untersucht werden kann.

Aufgabe der vorliegenden Erfindung ist es einen Weg aufzuzeigen, wie die Kosten zur Herstellung eines Lanzettenmagazins mit darin steril verpackten Lanzetten reduziert werden können. Ferner soll durch die vorliegende Erfindung eine einfachere Aufnahme und Untersuchung einer Körperflüssigkeitsprobe ermöglicht werden.

Diese Aufgabe wird durch ein Lanzettenmagazin mit den im Anspruch 1 angegebenen Merkmalen sowie durch ein Verfahren gemäß Anspruch 13 gelöst. Vorteilhafte Weiterbildungen der Erfindung sind Gegenstand von Unteransprüchen.

Im Rahmen der Erfindung wurde erkannt, dass für eine sterile Lagerung von Lanzetten in einem Magazin keine feuchtigkeitsdichte Verpackung erforderlich ist. Die Lanzetten eines erfindungsgemäßen Lanzettenmagazins sind in Kammern angeordnet, die mit einer gas- und feuchtigkeitsdurchlässigen Membran verschlossen sind. Die Membran eines erfindungsgemäßen Lanzettenmagazins verhindert unter Produktions-, Lager- und Gebrauchsbedingungen zwar ein Eindringen von Krankheitserregern, ist jedoch für ein sterilisierendes Gas, beispielsweise heißen Wasserdampf und/oder Ethylenoxid, durchlässig.

Produktions-, Lager- und Gebrauchsbedingungen sind in der Regel dadurch gekennzeichnet, dass die Membran bis zum Gewinnen einer Körperflüssigkeitsprobe keinen Flüssigkeiten ausgesetzt ist und nach der Sterilisation, bedingt durch das relativ geringe Volumen der Magazinkammer, nur geringen Gasdurchströmungen ausgesetzt ist. Dadurch ist gewährleistet, dass selbst Partikel, beispielsweise Mikroorganismen, die kleiner als die größten Membranporen sind, zuverlässig abgetrennt werden können. Als Faustformel für die Abtrennung von Partikeln aus einem langsamen Gasstrom kann in der Regel gelten, dass nur Partikel mit einem Durchmesser von weniger als einem Zehntel des maßgeblichen Porendurchmessers die Membran passieren können.

Die Poren bilden durch die Membran hindurch verlaufende Kanäle. Der relevante Porendurchmesser der Membran ist dabei durch den Kanaldurchmesser an seiner engsten Stelle bestimmt. Derjenige Kanal, welcher an seiner engsten Stelle den größten Durchmesser aufweist, bestimmt die Durchlässigkeit der Membran. Dieser Durchmesser ist der relevante Porendurchmesser.

Bevorzugt weist die erfindungsgemäß verwendete Membran durchgehende Poren mit einem relevanten Durchmesser von nicht mehr als 5 µm auf, besonders bevorzugt nicht mehr als 2 µm auf. Eine solche Membran kann somit Partikel mit einer Größe von 0,5 µm bzw. 0,2 µm zurückhalten. Mikroorganismen sind in der Regel größer und können deshalb von einer solchen Membran zurückgehalten werden. Asymmetrische Membranen können auf ihrer grobporigen Seite durchaus größere Poren aufweisen, die sich aber zu der feinporigen Seite hin verengen. Bei asymmetrischen Membranen ist der relevante Durchmesser für durchgehende Poren deshalb der Porendurchmesser der feinporigen Seite.

Während bei feuchtigkeitsdicht versiegelten Lanzettenmagazinen eine Sterilisation der darin enthaltenen Lanzetten nur mit erheblichem Aufwand durch Strahlung möglich ist, können Lanzetten in einem erfindungsgemäßen Lanzettenmagazin wesentlich kostengünstiger sterilisiert werden, indem ein sterilisierendes Gas durch die Membran in die Magazinkammer eingebracht wird. Dies ist mit geringem Aufwand möglich, beispielsweise indem Lanzettenmagazine für 20 Minuten 121°C heißem Wasserdampf ausgesetzt werden.

Gasdurchlässige Materialien können beispielsweise aus einem Fasermaterial hergestellt werden. Geeignet sind insbesondere Vliese aus Kunststofffasern. Für eine gasdurchlässige Membran können aber auch andere poröse Materialien, beispielsweise offenzellige Schaumstoffe, nach einem Phaseninversionsverfahren hergestellte Membranen oder ähnliches verwendet werden. Bevorzugt sind hydrophile Membranen.

Die Lanzetten eines erfindungsgemäßen Magazins haben eine Probenaufnahmeeinrichtung, beispielsweise einen oder mehrere Kanäle zur Aufnahme einer Körperflüssigkeitsprobe. Ein solcher Kanal ist bevorzugt als eine Rille ausgebildet, kann aber beispielsweise auch ein Schlitz sein. Derartige Probenaufnahmeeinrichtungen füllen sich beim Einstich der Lanzette in Körpergewebe durch Kapillarkräfte mit Körperflüssigkeit.

Überraschenderweise ermöglichen Lanzetten eines erfindungsgemäßen Lanzettenmagazins bei gleicher Geometrie der Probenaufnahmeeinrichtung eine wesentlich bessere Probenaufnahme als Lanzetten in feuchtigkeitsdicht versiegelten Magazinen, die durch Strahlungseinwirkung sterilisiert wurden. Dies wird darauf zurückgeführt, dass bei einer Sterilisation durch energiereiche Strahlung im Kunststoff eines Magazingehäuses Radikale entstehen und niedermolekulare Verbindungen freigesetzt werden, die in die Gasphase übertreten und sich im Laufe der Lagerzeit auf der Oberfläche einer Lanzette abscheiden können. Diese Abscheidungen scheinen eine hydrophobierende Wirkung auf die Oberfläche einer Lanzette zu haben und so die Probenaufnahme zu erschweren. Bei einem erfindungsgemäßen Lanzettenmagazin, dessen Lanzetten durch Einwirkung eines sterilisierenden Gases, insbesondere Wasserdampf, sterilisiert werden, wird die Oberfläche von Lanzetten dagegen nicht beeinträchtigt.

Nach der Sterilisation wird auf die gasdurchlässige Membran ein Testfeld mit Nachweisreagenzien zur Untersuchung einer Körperflüssigkeitsprobe aufgebracht. Geeignete Nachweisreagenzien sind bei handelsüblichen Teststreifen beispielsweise zur photometrischen oder elektrochemischen Messung der Konzentration von Glucose oder anderen medizinisch bedeutsamen Analyten bekannt. Indem bei einem erfindungsgemäßen Lanzettenmagazin Testfelder erst nach der Sterilisation angebracht werden, ist die Gefahr einer Beeinträchtigung empfindlicher Nachweisreagenzien durch den Sterilisationsvorgang ausgeschlossen.

Die gasdurchlässige Membran kann bei einem erfindungsgemäßen Magazin auf diese Weise sowohl als Sterilbarriere, die ein Eindringen von Krankheitskeimen verhindert, als auch zur Übergabe einer Körperflüssigkeitsprobe von einer Lanzette an das Testfeld verwendet werden. Indem nämlich eine gas- und flüssigkeitsdurchlässige Membran zum Verschließen einer Kammeröffnung verwendet wird, kann eine Körperflüssigkeitsprobe, die von der Probenaufnahmeeinrichtung einer Lanzette aufgenommen wurde, an das Testfeld weitergegeben werden. Hierfür genügt es, die Lanzette bzw. deren Probenaufnahmeeinrichtung mit der Innenseite der Membran in Kontakt zu bringen. Die Körperflüssigkeitsprobe kann dann durch Kapillarkräfte durch die Membran hindurch zu dem Testfeld befördert werden.

Um ein Testfeld mit Nachweisreagenzien zur Untersuchung einer Körperflüssigkeitsprobe auf die Oberseite der Membran aufzubringen werden, gibt es verschiedene Möglichkeiten. Beispielsweise kann das Testfeld auf einer Trägerfolie ausgebildet sein, die auf die Membran gelegt wird, so dass die Nachweisreagenzien zwischen der Trägerfolie und der gasdurchlässigen Membran angeordnet sind. Eine weitere Möglichkeit besteht darin, Nachweisreagenzien als Paste direkt auf die Oberfläche der Membran aufzutragen und so ein Testfeld auf der Oberseite der Membran auszubilden. Die Nachweisreagenzien können als eine einzige Schicht mit einer definierten Zusammensetzung vorliegen oder auch als mehrere Schichten, die sich in ihren jeweiligen Zusammensetzungen unterscheiden und funktional ergänzen. Beispielsweise kann eine Schicht der Nachweisreagenzien eine Reflexionsschicht ausbilden, während eine darüberliegende Schicht eine konzentrationsabhängige Verfärbung zeigt.

Bei einem erfindungsgemäßen Magazin weisen die Kammern zusätzlich zu der Stichöffnung, deren Verschluss bei einem Stich durchstoßen wird, eine weitere Öffnung auf die mit einer gas- und flüssigkeitsdurchlässigen Membran verschlossen ist, die mehrere Funktion erfüllt. Die Membran ermöglicht das Einbringen eines sterilisierenden Gases in die Kammer zum Sterilisieren der Lanzette, dient danach als Sterilbarriere, um ein Eindringen von Krankheitserregern in die Kammer zu verhindern, und transportiert nach einem Stich eine Körperflüssigkeitsprobe von der Probenaufnahmeeinrichtung einer Lanzette auf einer Seite der Membran zu einem Testfeld auf der anderen Seite der Membran. Die Membran hat also eine der Lanzette zugewandte Unterseite zur Aufnahme von Körperflüssigkeit von der Probenaufnahmeeinrichtung und eine Oberseite zur Übergabe von Körperflüssigkeit an ein auf der Membran liegendes Testfeld.

Bevorzugt hat jede Magazinkammer zusätzlich auch eine Einschuböffnung, deren Verschluss zum Auslösen eines Stichs von einem Stechgerät durchstoßen wird. Stichöffnung und Einschuböffnung können aber auch zu einer einzigen Öffnung zusammengefasst werden, die dann der Einfachheit halber ebenfalls als Stichöffnung bezeichnet wird.

Die vorliegende Erfindung betrifft also die Verwendung einer gas- und flüssigkeitsdurchlässigen Membran, die eine Öffnung einer Kammer eines Lanzettenmagazins verschließt, zum Transport einer Körperflüssigkeitsprobe von einer Lanzette auf einer Seite der Membran zu einem Testfeld auf der anderen Seite der Membran.

Die vorliegende Erfindung betrifft auch ein System zur Messung einer Analytkonzentration einer Körperflüssigkeitsprobe, mit einem Stechgerät und einem in das Stechgerät einsetzbaren Lanzettenmagazin, das ein Gehäuse aufweist und in sterilen Kammern eingeschlossene Lanzetten enthält, wobei wenigstens eine Öffnung jeder eine Lanzette enthaltenden Kammer mit einer gas- und flüssigkeitsdurchlässigen Membran verschlossen ist, die ein Testfeld trägt, und wobei das Stechgerät eine Mechanik enthält, um eine Stichbewegung einer Lanzette zu bewirken und die Probenaufnahmeeinrichtung einer Lanzette nach einem Stich mit der Innenseite der das Testfeld tragenden Membran in Kontakt zu bringen, so dass eine von der Probenaufnahmeeinrichtung aufgenommene Körperflüssigkeitsprobe durch Kapillarkräfte zu dem Testfeld bewegt werden kann.

Wie bereits erwähnt hat bei einem erfindungsgemäßen Lanzettenmagazin jede eine Lanzette enthaltende Magazinkammer wenigstens zwei, bevorzugt wenigstens drei, Öffnungen, nämlich eine Stichöffnung, deren Verschluss bei einem Stich von der Lanzette durchstochen wird, eine weitere Öffnung, die mit der flüssigkeitsdurchlässigen Membran verschlossen ist, die das Testfeld trägt, und bevorzugt auch eine Einschuböffnung, deren Verschluss von einem Antriebsmechanismus eines Stechgeräts durchstoßen wird, um einen Lanzette in eine Stichbewegung zu versetzten. Bevorzugt ist dabei nur die weitere Öffnung mit einer durchlässigen Membran verschlossen, während alle weiteren Kammeröffnungen mit einer gasundurchlässigen Siegelfolie verschlossen sind. Als Siegelfolie können insbesondere Kunststofffolien, mit Kunststoff beschichtete Metallfolien und Metallfolien verwendet werden.

Weitere Einzelheiten und Vorteile der Erfindung werden an einem Ausführungsbeispiel unter Bezugnahme auf die beigefügten Zeichnungen erläutert. Es zeigen:
- Figur 1: ein Ausführungsbeispiel eines erfindungsgemäßen Lanzettenmagazins;
- Figur 2: eine Hälfte des in Figur 1 dargestellten Magazins mit darin angeordneten Lanzetten; und
- Figur 3: eine Lanzette.

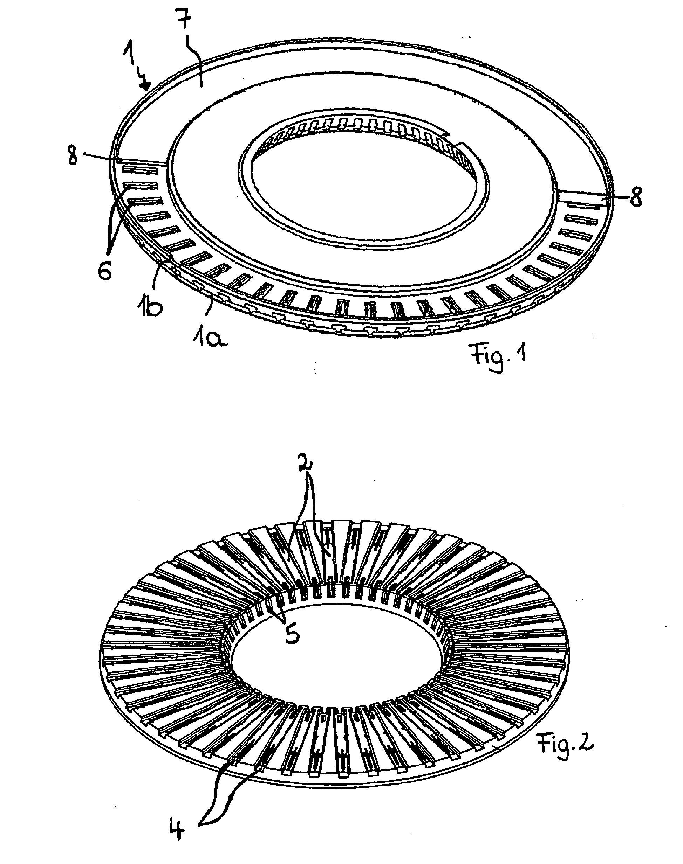

Figur 1 zeigt ein Lanzettenmagazin mit einem Kunststoffgehäuse, beispielsweise aus Polycarbonat oder Polysulfon. Bei dem dargestellten Ausführungsbeispiel ist das Gehäuse aus zwei Gehäusehälften 1 a, 1b zusammengefügt. Die untere Gehäusehälfte 1b ist in Figur 2 dargestellt. Das Gehäuse 1 hat mehrere Kammern, in denen jeweils eine Lanzette 2 angeordnet ist.

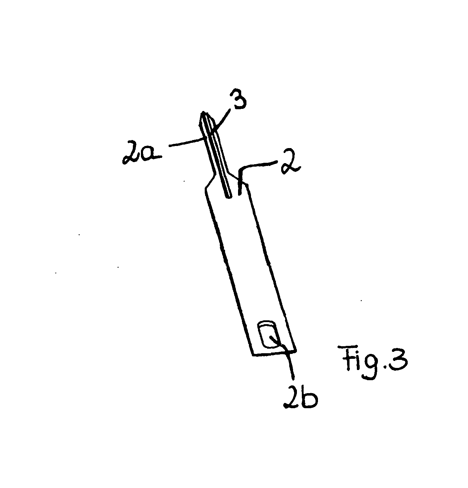

Ein Ausführungsbeispiel einer Lanzette 2 ist in Figur 3 dargestellt. Die Lanzette 2 hat eine Probenaufnahmeeinrichtung 3, die bei dem dargestellten Ausführungsbeispiel als ein Kanal, genauer gesagt eine Rille, ausgebildet ist. Bei einem Einstich in Körpergewebe eines Patienten füllt sich der Kanal durch Kapillarkräfte mit Blut und/oder interstitieller Flüssigkeit. Zur Vergrößerung der aufgenommenen Probenmenge können mehrere Kanäle nebeneinander vorgesehen sein. Die Probenaufnahmeeinrichtung 3 kann einen hydrophilen Belag aufweisen, beispielsweise Heparin, um die Probenaufnahme zu verbessern.

Die einzelnen Kammern des Gehäuses 1 haben bevorzugt jeweils wenigstens drei Öffnungen, nämlich eine Stichöffnung 4, aus der eine Lanzette 2 bei einem Stich austritt, eine Einschuböffnung 5, in die ein Stößel eines Stechgeräts eingeschoben werden kann, um eine Lanzette 2 für eine Stichbewegung aus der Stichöffnung 4 vorzuschieben und wieder zurückzuziehen, sowie eine weitere Öffnung 6, die von einem Testfeld 7 bedeckt ist. In Figur 1 ist nur die Hälfte der weiteren Öffnungen 6 bedeckt dargestellt, damit der Aufbau des Lanzettenmagazins besser erkennbar ist.

Die Öffnungen 6 sind mit einer flüssigkeitsdurchlässigen Membran 8 verschlossen, auf der das Testfeld 7 liegt. Die Membran 8 hat eine Durchlässigkeitsgrenze von weniger als 0,5 µm, bevorzugt 0,2 µm. Partikel, deren Größe die Durchlässigkeitsgrenze übersteigt werden von der Membran 8 abgehalten, während kleinere Partikel durch diese hindurch treten können. Die Poren der Membran 8 bilden durch die Membran 8 hindurchführende Kanäle. Die relevante Porengröße ist dabei der maximale Durchmesser der Kanäle an ihrer engsten Stelle. Die relevante Porengröße der Membran liegt bevorzugt bei höchstens 5 µm, bevorzugt höchstens 2 µm.

Geeignete Membranen 8 können beispielsweise aus Fasermaterial bestehen. Neben Papier sind insbesondere Materialien aus Kunststofffasern, insbesondere Vliese, geeignet. Unter der Marke "TYVEC ®" wird von der Firma DuPont ein geeignetes Vlies vertrieben, das aus verschweißten Polyethylenfasern besteht. Als Alternative zu Fasermaterialien können auch andere poröse Stoffe verwendet werden, beispielsweise Schaumstoffe oder nach dem Phaseninversionsverfahren gefertigte Membranen. Eine geeignete Membran 8 wird beispielsweise von der Firma Pall GmbH, Dreieich, Deutschland unter der Bezeichnung BTS 45 vertrieben. Besonders gut geeignet sind hydrophile Membranen.

Die übrigen Kammeröffnungen, bei dem dargestellten Beispiel also die Stichöffnung 4 und die Einschuböffnung 5, können an sich ebenfalls mit einer flüssigkeitsdurchlässigen und damit auch gasdurchlässigen Membran 8 verschlossen sein. Bevorzugt sind die Öffnungen 4, 5 aber mit kostengünstigerem Material, nämlich gasdichten Folien (nicht dargestellt) verschlossen. Geeignet sind insbesondere Kunststofffolien, Metallfolien und kunststoffbeschichtete Metallfolien.

Bei der Herstellung des in Figur 1 dargestellten Magazins werden zunächst die Lanzetten 2 in den Magazinkammern angeordnet. Anschließend werden die Kammeröffnung 4, 5 und 6 verschlossen. Danach werden die Lanzetten 2 sterilisiert, indem durch die gasdurchlässige Membran 8 hindurch ein sterilisierendes Gas in die Magazinkammern eingebracht wird. Beispielsweise kann das verschlossene Magazin für 20 Minuten 121°C heißem Wasserdampf ausgesetzt werden. Dem Wasserdampf können zur Verbesserung der Sterilisationswirkung weitere sterilisierende Gase beigemischt werden. Als sterilisierendes Gas kann beispielsweise auch Ethylenoxid verwendet werden, insbesondere gemäß DIN/ EN 550 oder EN ISO 11135.

Die Lanzetten 2 liegen in den Magazinkammern bloß, haben also Kontakt zu dem in den Kammern enthaltenen Gas und somit auch zu dem zur Sterilisation eingeleiteten Gas. Die Spitzen der Lanzetten 2 und ein an die Spitzen anschließender Stichbereich 2a, der bei einem Stich in den Körper eines Patienten eindringt, haben bevorzugt keinen Kontakt zu dem Magazingehäuse 1, so dass die gesamte Oberfläche des Stichbereichs 2a Kontakt zu dem in den Kammern enthaltenen Gas und somit auch zu dem zur Sterilisation eingeleiteten Gas hat. Der Stichbereich 2a kann deshalb besonders rasch durch Gaseinwirkung sterilisiert werden. Die Lanzetten 2 können in den Magazinkammern beispielsweise mit einem an den Stichbereich 2a anschließenden Lanzettenkörper gehalten werden und an Magazinwänden anliegen.

Nach der Sterilisation wird auf die gasdurchlässige Membran 8 ein Testfeld 7 mit Nachweisreagenzien zur Untersuchung einer Körperflüssigkeitsprobe aufgebracht. Die Nachweisreagenzien bewirken bei Anwesenheit eines Analyten, beispielsweise Glucose, eine Nachweisreaktion, durch deren Auswertung die gesuchte Analytkonzentration bestimmt werden kann. Bevorzugt werden Nachweisreagenzien verwendet, deren Nachweisreaktion eine Farbänderung des Testfeldes bewirkt, die eine fotometrische Auswertung ermöglicht, wie dies bei handelsüblichen Teststreifen gebräuchlich ist.

Die Kammeröffnungen 6 können jeweils mit einzelnen Membranplättchen verschlossen werden. Einfacher ist es jedoch, mehrere, bevorzugt alle, Kammeröffnungen 6 mit einem einzigen Stück Membran 8 zu bedecken. In entsprechender Weise können auf die verschlossenen Kammeröffnungen 6 jeweils einzelne Testfelder 7 aufgebracht werden. Einfacher ist es jedoch, eine Trägerfolie, beispielsweise aus transparentem Kunststoff, mit Nachweisreagenzien zu bedecken und auf die Membran 8 zu legen. Wenn eine undurchlässige Trägerfolie verwendet wird, ist darauf zu achten, dass die Reagenzschicht auf der Trägerfolie der Membran 8 zugewandt ist. Bevorzugt liegt die Reagenzschicht an der Membran 8 an, hat also mit dieser Kontakt, so dass eine Körperflüssigkeitsprobe gut von der Membran 8 in die Reagenzschicht übertreten kann.

Die gas- und flüssigkeitsdurchlässige Membran hat also eine der Lanzette 2 zugewandte Unterseite zur Aufnahme von Körperflüssigkeit von der Probenaufnahmeeinrichtung 3 und eine Oberseite zur Übergabe von Körperflüssigkeit an ein auf der Membran 8 liegendes Testfeld 7 aufweist. Bevorzugt hat die Membran an der Unterseite eine höhere Porengröße als an der Oberseite. Die erhöhte Porengröße an der Unterseite erleichtert den Übertritt von Körperflüssigkeit von der Lanzette in die Membran, während die reduzierte Porengröße an der Oberseite das Eindringen von Mikroorganismen und anderen Krankheitserregern erschwert.

Um nach einem Lanzettenstich einen guten Probenübertrag von der Probenaufnahmeeinrichtung 3 einer Lanzette 2 durch die Membran 8 hindurch zu dem Testfeld 7 zu ermöglichen, sollte die Reagenzschicht auf der Trägerfolie relativ glatt ausgebildet sein. Vorteilhaft sind insbesondere Rauhigkeitswerte SRq von weniger als 3 µm, bevorzugt weniger als 2 µm. Rauhigkeitswerte SRmax von weniger als 30 µm, insbesondere weniger als 20 µm sind günstig. Die angegebenen Rauhigkeitswerte SRmax und SRq beziehen sich jeweils auf Messungen mit einem Laser-Scanning-Mikroskop nach DIN EN ISO 25178/(2 und 3) und adäquaten Bildbearbeitungsfilter nach ISO/DIS 16610-21.

Das beschriebene Lanzettenmagazin kann in ein Magazinfach eines nicht dargestellten Stechgeräts eingesetzt werden, das einen Stechantrieb aufweist, um eine Lanzette 2 aus der Stichöffnung 4 herauszuschieben und wieder zurückzuziehen. Der Stechantrieb kann hierfür einen Stößel aufweisen, der bei einem Stich in eine Einschuböffnung 5 einer Magazinkammer eingeschoben wird, an eine Lanzette 2 ankoppelt und diese aus der Stichöffnung herausschiebt. Dabei werden sowohl eine die Einschuböffnung 5 verschließende Folie als auch eine die Stichöffnung 4 verschließende Folie durchstoßen. Um das Ankoppeln des Stechantriebs an die Lanzette 2 zu erleichtern kann diese ein Kopplungselement, bei dem dargestellten Ausführungsbeispiel einen Durchbruch 2b, aufweisen. Der Stechantrieb bewirkt, dass die Probenaufnahmeeinrichtung 3 einer Lanzette 2 am Ende einer Lanzettenbewegung an der Membran 8 anliegt. Eine aufgenommene Körperflüssigkeitsprobe wird dann durch Kapillarkräfte von der Membran 8 aufgenommen und durch diese hindurch zu dem Testfeld 7 transportiert. Ein geeigneter Stechantrieb kann beispielsweise als Rotorantrieb mit einer Kulissensteuerung realisiert werden. Zusätzlich kann das Stechgerät eine Messeinrichtung haben, die zusammen mit einem Testfeld 7 eine Konzentrationsbestimmung, beispielsweise durch eine fotometrische Messung, ermöglicht.

### Bezugszahlen

- 1: Gehäuse
- 1 a: Gehäusehälfte
- 1b: Gehäusehälfte
- 2: Lanzette
- 2a: Stichbereich
- 2b: Durchbruch
- 3: Probenaufnahmeeinrichtung
- 4: Stichöffnung
- 5: Einschuböffnung
- 6: weitere Öffnungen
- 7: Testfeld
- 8: Membran

## Patentansprüche

1. Lanzettenmagazin mit einem Gehäuse (1) und mehreren Lanzetten (2), die jeweils in einer sterilen Kammer des Gehäuses (1) eingeschlossen sind, wobei die Kammern jeweils eine Stichöffnung (4) aufweisen, die mit einer Folie verschlossen sind,
**dadurch gekennzeichnet, dass**
die Lanzetten (2) eine Probenaufnahmeeinrichtung (3) zur Aufnahme von Körperflüssigkeit aufweisen, und
die Kammern jeweils eine weitere Öffnung (6) aufweisen, die mit einer gas- und flüssigkeitsdurchlässigen Membran (8) verschlossen ist, die eine der Lanzette (2) zugewandte Unterseite zur Aufnahme von Körperflüssigkeit von der Probenaufnahmeeinrichtung (3) und eine Oberseite zur Übergabe von Körperflüssigkeit an ein auf der Membran (8) angeordnetes Testfeld (7) aufweist.

2. Lanzettenmagazin nach einem der vorstehenden Ansprüche, **dadurch gekennzeichnet, dass** die Membran (8) aus Polymermaterial hergestellt ist.

3. Lanzettenmagazin nach Anspruch 2, **dadurch gekennzeichnet, dass** die Membran (8) nach einem Phaseninversionsverfahren hergestellt ist.

4. Lanzettenmagazin nach einem der vorstehenden Ansprüche, **dadurch gekennzeichnet, dass** die Membran (8) hydrophil ist.

5. Lanzettenmagazin nach einem der vorstehenden Ansprüche, **dadurch gekennzeichnet, dass** die Membran (8) an der Unterseite größere Poren als an der Oberseite aufweist.

6. Lanzettenmagazin nach einem der vorstehenden Ansprüche, **dadurch gekennzeichnet, dass** die gasdurchlässige Membran (8) ein Blatt ist, das mehrere Kammeröffnungen (6) bedeckt.

7. Lanzettenmagazin nach einem der vorstehenden Ansprüche, **dadurch gekennzeichnet, dass** die Lanzettenspitzen Kontakt zu in den Kammern enthaltenem Gas haben.

8. Lanzettenmagazin nach einem der vorstehenden Ansprüche, **dadurch gekennzeichnet, dass** die Probenaufnahmeeinrichtung (3) einen Kanal aufweist.

9. Lanzettenmagazin nach einem der vorstehenden Ansprüche, **dadurch gekennzeichnet, dass** Nachweisreagenzien des Testfeldes (7) die Membran (8) kontaktieren.

10. Lanzettenmagazin nach einem der vorstehenden Ansprüche, **dadurch gekennzeichnet, dass** das Testfeld (7) auf einer Trägerfolie ausgebildet ist, wobei die Nachweisreagenzien zwischen der Trägerfolie und der gasdurchlässigen Membran (8) angeordnet sind.

11. Lanzettenmagazin nach Anspruch 10, **dadurch gekennzeichnet, dass** die Trägerfolie mehrere Öffnungen (6) bedeckt.

12. Lanzettenmagazin nach einem der vorstehenden Ansprüche, **dadurch gekennzeichnet, dass** die Stichöffnung (4) mit einer gasundurchlässigen Folie verschlossen ist.

13. Verfahren zum Herstellen eines Lanzettenmagazins, wobei in Kammern eines Magazingehäuses (1) jeweils eine Lanzette (2) angeordnet wird und die Kammern anschließend verschlossen werden, **dadurch gekennzeichnet, dass** wenigstens eine Öffnung (4, 5, 6) jeder eine Lanzette (2) enthaltenden Kammer mit einer gasdurchlässigen Membran (8) verschlossen wird und danach ein sterilisierendes Gas durch die Membran (8) hindurch in die Kammer eingebracht wird, indem das verschlossene Magazingehäuse (1) dem sterilisierenden Gas, vorzugsweise Wasserdampf und/oder Ethylenoxid, ausgesetzt wird.

14. Verfahren nach Anspruch 13, **dadurch gekennzeichnet, dass** nach der Sterilisation auf die Membran (8) ein Testfeld (7) mit Nachweisreagenzien zur Untersuchung einer Körperflüssigkeitsprobe aufgebracht wird.

15. Verwendung einer gas- und flüssigkeitsdurchlässigen Membran (8), die eine Öffnung (6) einer Kammer eines Lanzettenmagazins verschließt, zum Transport einer Körperflüssigkeitsprobe von einer Lanzette auf einer Seite der Membran zu einem Testfeld auf der anderen Seite der Membran.

## Claims

1. A lancet magazine comprising a housing (1) and a plurality of lancets (2) each of which is enclosed in a sterile chamber of the housing (1), wherein the chambers each have a puncturing opening (4) which is closed with a foil, **characterized in that**
the lancets (2) comprise a sample receiving device (3) for receiving body fluid and
the chambers each comprise a further opening (6) which is closed with a membrane (8) that is permeable to gas and fluid and comprises a lower side that faces the lancet (2) and serves to receive body fluid from the sample receiving device (3) and an upper side that serves to transfer body fluid to a test field (7) arranged on the membrane (8).

2. The lancet magazine according to any one of the preceding claims, **characterized in that** the membrane (8) is produced from polymer material.

3. The lancet magazine according to claim 2, **characterized in that** the membrane (8) is produced according to a phase inversion method.

4. The lancet magazine according to any one of the preceding claims, **characterized in that** the membrane (8) is hydrophilic.

5. The lancet magazine according to any one of the preceding claims, **characterized in that** the membrane (8) comprises pores on the lower side the size of which is in excess of that of the pores on the upper side.

6. The lancet magazine according to any one of the preceding claims, **characterized in that** the gas-permeable membrane (8) is a sheet that covers a plurality of chamber openings (6).

7. The lancet magazine according to any one of the preceding claims, **characterized in that** the lancet tips are in contact with a gas contained in the chambers.

8. The lancet magazine according to any one of the preceding claims, **characterized in that** the sample receiving device (3) comprises a channel.

9. The lancet magazine according to any one of the preceding claims, **characterized in that** analytical reagents of the test field (7) are in contact with the membrane (8).

10. The lancet magazine according to any one of the preceding claims, **characterized in that** the test field (7) is formed on a backing film, wherein the analytical reagents are arranged between the backing film and the gas-permeable membrane (8).

11. The lancet magazine according to claim 10, **characterized in that** the backing film covers a plurality of openings (6).

12. The lancet magazine according to any one of the preceding claims, **characterized in that** the puncturing opening (4) is closed with a foil that is impermeable to gas.

13. A method for the production of a lancet magazine, wherein a lancet (2) is arranged in each of the chambers of a magazine housing (1) and the chambers are closed thereafter, **characterized in that** at least one opening (4, 5, 6) of each chamber containing a lancet (2) is closed with a gas-permeable membrane (8) and a sterilizing gas is subsequently introduced through the membrane (8) and into the chamber by exposing the closed magazine housing (1) to the sterilizing gas, preferably water vapor and/or ethylene oxide.

14. The method according to claim 13, **characterized in that**, after sterilization, a test field (7) with analytical reagents for the examination of a body fluid sample is applied onto the membrane (8).

15. A use of a membrane (8) that is permeable to gas and fluid and closes an opening (6) of a chamber of a lancet magazine, in order to transport a body fluid sample from a lancet on one side of the membrane to a test field on the other side of the membrane.

## Revendications

1. Cartouche de lancettes comprenant un boîtier (1) et plusieurs lancettes (2) enfermées chacune dans une chambre stérile du boîtier (1), chaque chambre présentant un orifice de piqûre (4) qui est obturé par une feuille, **caractérisée en ce que**
lesdites lancettes (2) comportent un dispositif de réception d'échantillon (3) pour la réception d'un liquide corporel, et
lesdites chambres présentent chacune un autre orifice (6) qui est obturé par une membrane (8) perméable au gaz et au liquide, ayant une face inférieure tournée vers la lancette (2) pour recevoir le liquide corporel du dispositif de réception d'échantillon (3) et une face supérieure pour transférer le liquide corporel vers une zone réactive (7) disposée sur la membrane (8).

2. Cartouche de lancettes selon l'une des revendications précédentes, **caractérisée en ce que** la membrane (8) est réalisée en matériau polymère.

3. Cartouche de lancettes selon la revendication 2, **caractérisée en ce que** la membrane (8) est réalisée selon un procédé d'inversion de phase.

4. Cartouche de lancettes selon l'une des revendications précédentes, **caractérisée en ce que** la membrane (8) est hydrophile.

5. Cartouche de lancettes selon l'une des revendications précédentes, **caractérisée en ce que** la membrane (8) présente des pores plus grands sur la face inférieure que sur la face supérieure.

6. Cartouche de lancettes selon l'une des revendications précédentes, **caractérisée en ce que** la membrane (8) perméable au gaz est une feuille recouvrant plusieurs orifices (6) de chambres.

7. Cartouche de lancettes selon l'une des revendications précédentes, **caractérisée en ce que** les pointes de lancettes sont en contact avec le gaz contenu dans les chambres.

8. Cartouche de lancettes selon l'une des revendications précédentes, **caractérisée en ce que** le dispositif de réception d'échantillon (3) comporte un canal.

9. Cartouche de lancettes selon l'une des revendications précédentes, **caractérisée en ce que** les réactifs de détection de la zone réactive (7) entrent en contact avec la membrane (8).

10. Cartouche de lancettes selon l'une des revendications précédentes, **caractérisée en ce que** la zone réactive (7) est réalisée sur une feuille support, les réactifs de détection étant disposés entre ladite feuille support et la membrane (8) perméable au gaz.

11. Cartouche de lancettes selon la revendication 10, **caractérisée en ce que** la feuille support recouvre plusieurs orifices (6).

12. Cartouche de lancettes selon l'une des revendications précédentes, **caractérisée en ce que** l'orifice de piqûre (4) est obturé par une feuille imperméable au gaz.

13. Procédé de fabrication d'une cartouche de lancettes où on place une lancette (2) dans chaque chambre d'un boîtier de cartouche (1) puis on obture les chambres, **caractérisé en ce que** l'on obture au moins un orifice (4, 5, 6) de chaque chambre contenant une lancette (2) par une membrane (8) perméable au gaz, puis on introduit un gaz stérilisant dans la chambre à travers ladite membrane (8), en exposant le boîtier de cartouche (1) obturé audit gaz stérilisant, qui est de préférence de la vapeur d'eau et/ou de l'oxyde d'éthylène.

14. Procédé selon la revendication 13, **caractérisé en ce que**, après la stérilisation, on dépose sur la membrane (8) une zone réactive (7) contenant des réactifs de détection pour l'analyse d'un échantillon de liquide corporel.

15. Utilisation d'une membrane (8) perméable au gaz et au liquide obturant un orifice (6) d'une chambre d'une cartouche de lancettes, pour transporter un échantillon de liquide corporel d'une lancette sur l'une des faces de la membrane vers une zone réactive sur l'autre face de la membrane.
